# FASCICULE DE BREVET EUROPEEN

(11) **EP 2 356 444 B1**
(45) Date de publication et mention de la délivrance du brevet: **13.02.2013**
(21) Numéro de dépôt: 09801467.3
(22) Date de dépôt: 04.12.2009
(51) Int. Cl.: G01N 33/24

(54) **PROCEDE DE DETERMINATION DE LA TEMPERATURE MAXIMALE SUBIE PAR UNE ROCHE SEDIMENTAIRE NOTAMMENT DANS LES DOMAINES DE FORMATION DES HYDROCARBURES**
VERFAHREN ZUR BESTIMMUNG DER VON EINEM SEDIMENTGESTEIN ERREICHTEN HÖCHSTTEMPERATUR, IM BESONDEREN BEI KOHLENWASSERSTOFFFORMATIONSFELDERN
METHOD FOR DETERMINING THE MAXIMUM TEMPERATURE REACHED BY A SEDIMENTARY ROCK, IN PARTICULAR IN THE HYDROCARBON FORMATION FIELDS

(30) Priorité: 10.12.2008 FR 0806921
(43) Date de publication de la demande: 17.08.2011
(73) Titulaire: Pozzi, Jean Pierre, 75018 Paris (FR); Aubourg, Charles, 38500 Voiron (FR)
(72) Inventeur: Pozzi, Jean Pierre, 75018 Paris (FR); Aubourg, Charles, 38500 Voiron (FR)
(74) Mandataire: Demulsant, Xavier
(86) Numéro de dépôt international: PCT/FR2009/001391
(87) Numéro de publication internationale: WO 2010/066961

(56) Documents cités:
- DUNLOP, DJ; ÖZDEMIR,Ö; CLARK, DA; SCHMIDT, PW: "Time-temperature relations for the remagnetization of pyrrhotite (Fe7S8) and their use in estimating paleotemperatures" EARTH AND PLANETARY SCIENCE LETTERS, vol. 176, 2000, pages 107-116, XP002538742
- ROCHETTE, P; FILLION, G; MATTEI, J-L; DEKKERS, MJ: "Magnetic transition at 30-34 Kelvin in pyrrhotite: insight into a widespread occurrence of this mineral in rocks" EARTH AND PLANETARY SCIENCE LETTERS, vol. 98, 1990, pages 319-328, XP002538743
- SCHILL, E; APPEL, E; GAUTAM, P: "Towards pyrrhotite/magnetite geothermometry in low-grade metamorphic carbonates of the Tethyan Himalayas (Shiar Khola, Central Nepal)" JOURNAL OF ASIAN EARTH SCIENCES, vol. 20, no. 3, 2002, pages 195-201, XP002538776
- RUNCORN, S.K.: "Application of the remanent magnetization of rocks - Based on a lecture to the 7th meeting of the European Association of Exploration Geophysicists, held in the Hague, December 1954" GEOPHYSICAL PROSPECTING, vol. 4, no. 3, 27 avril 2006 (2006-04-27), pages 221-225, XP002538744

## Description

La présente invention se rapporte à une méthode de détermination de la température maximale subie par une roche sédimentaire pendant un chauffage géologique par exemple lié à l'enfouissement. Il s'agit particulièrement des sédiments propres à être le siège de la formation ou de stockage d'hydrocarbures. Plus particulièrement, cette invention est relative à des méthodes qui caractérisent des minéraux magnétiques par certaines de leurs propriétés magnétiques.

Il est bien connu que certains composés du fer, principalement les oxydes et les sulfures, montrent des caractéristiques magnétiques permettant leur identification. Le principe sous-jacent sur lequel repose la présente invention est que des sulfures et des oxydes de fer, sont modifiés, ou créés, par des chauffages même très modérés (environ 60°C à 230°C) , et que cela se traduit dans des modifications de leurs propriétés magnétiques.

La gamme de température qui couvre de 60°C à 230°C environ est déterminante pour prévoir les conditions de maturation de la matière organique, et donc le type de dépôt minéral. Les transformations mises en jeu au cours de l'impact thermique impliquent des teneurs extrêmement faibles des minéraux transformés et/ou créés, et les méthodes magnétiques sont parfaitement adaptées pour le dosage de ces minéraux. De la détermination de la température maximale subie par une pluralité d'échantillons prélevés dans une formation, on déduit la température maximale subie par la partie échantillonnée de cette formation. Ce principe est connu, voir Dunlop et al. Earth Planet. Sci. Lett. 176 (2000) 107-116 et Rochette et al. Earth Planet. Sci. Lett. 98 (1990) 319-328. Dans la suite de l'exposé, le procédé est dénommé géothermomètre destiné à déterminer une paléo-température.

Dans la gamme des paléo-températures qui intéresse l'industrie pétrolière, plusieurs techniques sont utilisées.

Le Rock-Eval est la technique la plus utilisée dans l'industrie pétrolière. Cette technique permet, par chauffage de l'échantillon (jusqu'à 850°C) , de trouver les caractéristiques de la matière organique qu'il contient. Il ressort de cette analyse une température Tmax (400°C à 700°C) , qui est indicative du degré de maturité de la matière organique. Cette température maximale permet de savoir où se situe la roche par rapport aux fenêtres à huile et à gaz. Cependant, Le Rock-Eval ne donne pas une paléo-température absolue; le Tmax étant une température de pyrolyse. La paléo-température est établie en contraignant la cinétique thermique des échantillons. Le Rock- Eval est une méthode destructive, ne s'appliquant que sur des roches où la teneur en matière organique excède 100 parties par million (ppm). Le Rock-Eval ne s'applique en principe que sur les roches mères.

Il existe des méthodes optiques, comme la réflectance de la vitrinite, le degré d'altération des conodontes ou des acritarches, qui permettent de calibrer qualitativement le degré de maturation de la matière organique, et de situer cette dernière par rapport à la fenêtre à huile. La réflectance de la vitrinite implique la présence de matière organique d'origine ligno-cellulosique issue de végétaux supérieurs et donc absente des séries marines souvent à l'origine du pétrole. Les conodontes et les acritarches sont des fossiles qui ne se rencontrent pas dans toutes les séries sédimentaires. Des méthodes magnétiques ont été décrites, basées sur l'étude des aimantations rémanentes produites dans le sédiment par le chauffage géologique et le refroidissement. Ces réaimantations se composent vectoriellement avec les aimantations préexistantes et elles restent difficiles à isoler.

L'invention a donc pour but de remédier à tout ou partie des inconvénients précités. Elle se propose de donner une évaluation de la température maximale atteinte par la formation sédimentaire, d'une manière rapide, sur quelques milligrammes de roche, et de façon non destructive. L'invention s'applique sur des formations sédimentaires de type roches mères contenant de la matière organique, mais aussi sur d'autres formations par exemple du type de roches réservoirs (carbonates, roches détritiques). Ces sédiments seront désignés dans le texte suivant par -les sédiments-.

Il est, par ailleurs, connu que la magnétite (Fe3O4) apparaît dans les sédiments pour une température de chauffage d'environ 100°C.

L'invention s'appuie sur les découvertes suivantes :
1- la découverte de l'apparition, dans les sédiments, de la pyrrhotite (Fe7S8) à partir d'une température de chauffage aussi basse que 60°C environ. Cette pyrrhotite en grains fins et à très faible teneur sera appelée -pyrrhotite P- dans la suite de l'exposé.
2- la découverte d'une transition magnétique caractéristique de la dite pyrrhotite P pour une température proche de 35 K. Cette transition sera appelée -Transition P- dans la suite de l'exposé.
3- la découverte d'un mode opératoire spécifique pour la mise en évidence de la dite transition à basse température jusqu'à quelques kelvin

L'invention décrit le procédé selon lequel la combinaison des signatures magnétiques de la pyrrhotite P et de la magnétite est une fonction de la température de réchauffement géologique du sédiment et constitue un géothermomètre dont on peut établir une calibration.

D'une façon générale, l'analyse de l'évolution de l'aimantation d'une roche lors d'un refroidissement jusqu'à une température de quelques kelvin permet de mettre en évidence et de reconnaître les minéraux magnétiques courants par les transitions qu'ils subissent.

Les deux minéraux magnétiques (Fe7S8 et Fe3O4) , sont à des concentrations de l'ordre de la centaine de parties par million, avec des tailles de grains nanométriques à micrométriques. Ces minéraux ne sont donc pas facilement détectables avec les méthodes d'investigation habituelles disponibles dans l'industrie pétrolière. Les méthodes d'analyse magnétiques, et particulièrement celles à basses températures, permettent la caractérisation des minéraux magnétiques transformés à des concentrations de quelques dizaines de parties par milliard.

Pour analyser ces transitions caractéristiques, on peut utiliser par exemple, et sans que cela soit limitatif, l'évolution d'une Aimantation Rémanente Isotherme dénommée dans la suite ARI, de préférence à saturation. Cette ARI peut être produite par l'application d'un champ magnétique continu intense, par exemple de 2 Tesla ou plus. L'ARI peut être acquise à température ambiante et son intensité est suivie pendant le refroidissement de l'échantillon. L'ARI peut être produite à basse température et son intensité est suivie pendant le réchauffement de l'échantillon. Dans les exemples donnés, on suit successivement la variation d'une ARI acquise à température ordinaire pendant le refroidissement jusqu'à 10K par exemple puis la variation jusqu'à l'ambiante d'une ARI acquise à 10K dans le même champ.

On pourrait aussi mettre en évidence les transitions des minéraux investigués par le suivi, pour les mêmes très basses températures, de la susceptibilité magnétique du sédiment, mesurée soit en champs magnétiques alternatifs, éventuellement en utilisant plusieurs fréquences, soit en champs magnétiques continus.

Plus précisément, l'identification de la pyrrhotite P en grains très fins et à des très faibles teneurs, par sa transition P spécifique, repose sur un mode opératoire nouveau qui consiste à appliquer, au moins pendantle refroidissement, un champ magnétique continu faible, de l'ordre de 5 micro tesla par exemple. Sans l'application de ce champ continu faible au moins pendant le refroidissement jusqu'à 10K par exemple, la transition P peut ne pas être mise en évidence.

D'autres caractéristiques et avantages de l'invention apparaîtront à l'examen de la description détaillée ci-après, et des dessins annexés donnés à titre d'exemples non limitatifs.
La figure 1 présente la signature magnétique d'un échantillon d'argilites naturelles Aalénienne du Jura suisse. Il est visualisé la courbe de refroidissement d'une ARI acquise à la température de 300 K.
La figure 2 présente la signature magnétique d'un échantillon d'argilites naturelles Aalénienne du Jura suisse par ailleurs présenté dans la figure 1. Il est visualisé la courbe de réchauffement d'une ARI acquise à la température de 10 K.
La figure 3 présente un exemple de calibration d'un géothermomètre. Il est visualisé l'évolution de deux paramètres, Prec et Pref, déduits de courbes similaires à celles présentées dans les figures 1 et 2.

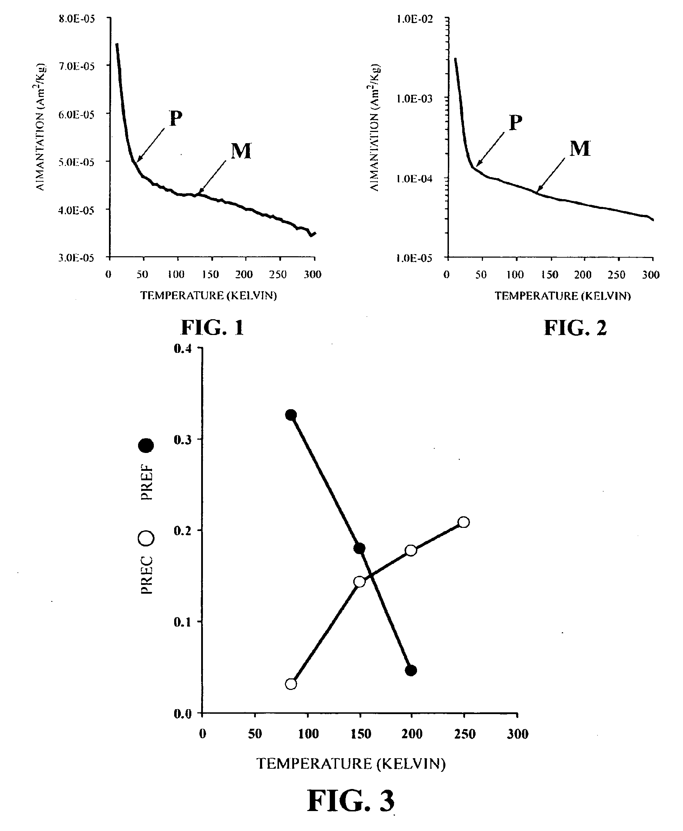

On se réfère tout d'abord à la Figure 1 où la courbe représente l'évolution, pendant un refroidissement d'une ARI acquise à la température ordinaire sous un champ magnétique de 2,5 Tesla et appelée ARI_{300K}. Les températures sont données en abscisse, les aimantations, en ordonnées, sont en Am²/Kg Le refroidissement a lieu sous un champ magnétique faible, de 5 microtesla. L'échantillon étudié est une argilite Aalénienne du Jura suisse ayant subi une température maximale de 85°C environ sous l'effet de son enfouissement au cours de son histoire géologique. Une transition à 120K (dite transition de Verwey) indique la présence de magnétite, notée M sur la courbe. L'augmentation de l'aimantation à partir de 35K environ est caractéristique de la transition P, notée P sur la courbe. La transition P, peut être caractérisée, par exemple, par l'amplitude de l'augmentation de l'aimantation entre la température de 35K et celle de 10K, soit (ARI_{300K}(10K)-ARI_{300K}(35K))/ARI_{300K}(10K) où la première température, en indice, indique la température d'acquisition de l'ARI et la seconde, entre parenthèses, indique la température de la mesure.

Dans la figure 2, la courbe représente l'évolution lors d'un réchauffement de 10K à 300K, d'une ARI acquise dans le même champ de 2.5T à la température de 10K et appelée ARI_{10K}. L'échantillon est le même que celui de la figure 1. La température en kelvin est donnée en abscisse et l'aimantation en Am²/Kg est représentée en ordonnée selon une échelle logarithmique. La transition de Verwey, notée M sur la courbe, indique la présence de la magnétite à 120K. La diminution de l'aimantation de 10K à 35K est une autre caractéristique de la transition P, notée P sur la courbe. La transition P, peut donc aussi être caractérisée, par exemple, par l'amplitude de la diminution de l'aimantation entre la température de 10K et celle de 35K soit ARI_{10K}(35K)/ ARI_{10K}(10K) où la première température, en indice, indique la température d'acquisition de l'ARI et la seconde, ) entre parenthèses, indique la température de la mesure.

On note que les variations d'aimantation montrée dans la figure 1 et la figure 2 sont, pour chaque température, principalement le résultat de la combinaison des aimantations de la pyrrhotite et de la magnétite sans qu'on puisse exclure les contributions moindres d'autres minéraux.

On définit ainsi deux paramètres qui peuvent servir à caractériser les transitions magnétiques survenues dans l'expérience décrite figure 1. Ces paramètres sont donnés à titre d'exemples non limitatifs et d'autres paramètres caractéristiques des transitions ou des concentrations en pyrrhotite et magnétite, pourraient être utilisés également:
De la courbe de refroidissement de 1' ARI_{300K}, figure 1:
   Pref= (ARI_{300K}(10K)-ARI_{300K}(35K))/ARI_{300K}(10K)
De la courbe de réchauffement de l' ARI_{10K}, figure 2:
   Prec= ARI_{10K}(35K)/ARI_{10K}(10K)

On se réfère maintenant à la figure 3 qui représente une calibration du géothermomètre basée sur les variations des paramètres Pref et Prec en fonction des températures de réchauffement en laboratoire déterminés sur le même sédiment. Les chauffages sont effectués en capsules d'or, scellées. Les températures de chauffage, 150°C, 200°C et 250°C, sont en abscisse, les rapports Prec et Pref mesurés sont en ordonnée. On donne également les paramètres mesurés sur l'échantillon du sédiment naturel chauffé au cours de son histoire géologique et que l'on a présenté dans la figure 1 et la figure 2. Les valeurs de Pref et de Prec sont consignées dans la Table. On remarquera qu'il n'y a pas de valeur de Prec à 250°C car la transition P n'a pas été détectée dans la courbe de refroidissement de l'ARI_{300K}. L'invention propose que les rapport Prec et Pref, ou que tout autre rapport déduit d'autres paramètres qui donnerait une bonne estimation des contributions de la magnétite et de la pyrrhotite, fournit une estimation de la température maximale subie par le sédiment pour servir de géothermomètre dans la gamme de température allant de 60°C à 230°C environ. Les rapports Prec et Pref, ainsi que consignés dans la Table, peuvent servir d'exemple d'étalonnage du géothermomètre. La température atteinte par la roche à l'état naturel est tirée de la littérature et elle est donnée à 20% près. Les températures de laboratoire sont précises au degré près.

**Table**

| Echantillon d'argilites | naturelle | Labo | Labo | Labo |
|---|---|---|---|---|
| Température | 85°C | 150°C | 200°C | 250°C |
| Prec | 0,03 | 0,14 | 0,18 | 0,21 |
| Pref | 0,33 | 0,18 | 0,05 | - |

Ces valeurs d'étalonnage donnent la température maximale, ou paléo-température, subie par un échantillon de la formation géologique étudiée plus généralement par un échantillon d'argilites, par comparaison avec les rapports Prec et Pref que donne la mesure effectuée sur cet échantillon selon l'exemple de réalisation du procédé décrit ci-avant. On estime alors que cette paleo-température est donnée à 15°C près environ. Il n'est pas exclu que des corrections doivent être apportées à ces valeurs d'étalonnage pour des familles de roches différentes telles que des calcaires marneux ou silteux, par exemple. Ces corrections peuvent être établies par chauffage en laboratoire ou par toute autre méthode qui pourrait donner des repères de température.

Si une plus grande précision est nécessaire, par exemple lorsqu'on veut suivre l'évolution de la paléo-température d'échantillons prélevés dans un forage, on décrit ici un étalonnage spécifique. Après la mesure des coefficients Prec et Pref et la détermination de la paléo-température d'un échantillon, on peut chauffer de nouveau, en laboratoire le dit échantillon à des températures légèrement inférieures et/ou légèrement supérieures à la paléo-température trouvée, par exemple supérieure de 10°C. Une nouvelle mesure selon le procédé est faite. Prec et Pref conduisent à une nouvelle température trouvée qui doit être égale à la température à laquelle on a chauffé l'échantillon en laboratoire si cette dernière est supérieure à la paléo-température. Si, par contre, la température de réchauffement trouvée reste égale à la paléo-température on en déduit que cette paléo-température était légèrement sous estimée. On peut ou non affiner encore la mesure de la paléo-température. Ce chauffage peut avantageusement avoir lieu dans un compartiment approprié, au même endroit que la mesure des transitions magnétiques à très basse température jusqu'à quelques kelvin par exemple, sans sortir l'échantillon du porte échantillon amagnétique, adapté aux températures d'utilisation.

L'invention ne se limite pas à l'exemple de réalisation du procédé décrit ci-avant seulement à titre d'exemple, mais elle englobe les variantes que pourra envisager l'homme de l'art dans le cadre des revendications ci-après.

## Revendications

1. Procédé de détermination de la température maximale subie par un échantillon de roche sédimentaire lors d'un chauffage naturel pendant un processus géologique, dans la gamme de 60°C à 230°C environ, **caractérisé en ce qu'**il comprend les étapes suivantes:
a. Analyse à très basse température, jusqu'à quelques kelvin, jusqu'à 10 kelvin par exemple, des transitions magnétiques destinées à caractériser un oxyde de fer et un sulfure de fer formés ou détruits dans l'échantillon par le chauffage géologique du sédiment.
b. Détermination de la température maximale subie par l'échantillon, à l'aide d'une calibration obtenue par la mesure des mêmes paramètres magnétiques sur le même sédiment ou sur d'autres sédiments de même nature, chauffés préalablement à des températures connues, en laboratoire.

2. Procédé selon la revendication 1, **caractérisé en ce que** l'oxyde de fer et le sulfure de fer sont la magnétite et la pyrrhotite.

3. Procédé selon l'une des revendications 1 et 2 **caractérisé en ce que** les transitions mesurées sont les transitions magnétiques caractéristiques de la magnétite et de la pyrrhotite et qui se produisent pour des températures fixes situées entre la température ambiante environ et quelques kelvin, jusqu'à 10K par exemple.

4. Procédé selon l'une des revendications 1 à 3 **caractérisé en ce que** les transitions magnétiques sont mises en évidence en suivant l'évolution, d'une aimantation rémanente isotherme artificielle produite par un champ magnétique continu assez fort pour, si possible, saturer magnétiquement au moins la magnétite et la pyrrhotite.

5. Procédé selon l'une des revendications 1 à 4 **caractérisé en ce qu'**un champ magnétique continu de faible intensité, par exemple de l'ordre de 5 micro tesla, est appliqué au moins pendant le refroidissement jusqu'à quelques kelvin, jusqu'à 10K par exemple.

6. Procédé selon l'une des revendications 1 à 5 **caractérisé en ce que** le rôle du champ magnétique continu de faible intensité appliqué, au moins pendant le refroidissement jusqu'à quelques kelvin, jusqu'à 10K par exemple, est de mettre en évidence une nouvelle transition de la pyrrhotite appelé transition P.

7. Procédé selon l'une des revendications 1 à 3 **caractérisé en ce que** les transitions magnétiques sont également mises en évidence en suivant l'évolution de la susceptibilité magnétique de l'échantillon pour des basses températures jusqu'à quelques degrés K.

## Claims

1. Method of determining the maximum temperature undergone by a specimen of sedimentary rock during natural heating over a geological process, within the range from 60°C to 230°C approximately, **characterized in that** it comprises the following steps:
a. analysis at very low temperature, down to a few kelvin, for example down to 10 kelvin, of the magnetic transitions intended to characterize an iron oxide and an iron sulfide that are formed or destroyed in the specimen by the geological heating of the sediment; and
b. determination of the maximum temperature undergone by the specimen, using a calibration obtained by measuring the same magnetic parameters on the same sediment or on other sediments of the same nature, heated beforehand in the laboratory to known temperatures.

2. Method according to Claim 1, **characterized in that** the iron oxide is magnetite and the iron sulfide is pyrrhotite.

3. Method according to either of Claims 1 and 2, **characterized in that** the measured transitions are magnetic transitions characteristic of magnetite and pyrrhotite and which occur at fixed temperatures lying between approximately room temperature and a few kelvin, for example down to 10 K.

4. Method according to one of Claims 1 to 3, **characterized in that** the magnetic transistions are detected by monitoring the variation in an artificial isothermal remanent magnetization produced by quite a high DC magnetic field so as, if possible, for at least the magnetite and the pyrrhotite to be magnetically saturated.

5. Method according to one of Claims 1 to 4, **characterized in that** a low-intensity DC magnetic field, for example of the order of 5 microtesla, is applied at least during the cooling down to a few Kelvin, for example down to 10 K.

6. Method according to one of Claims 1 to 5, **characterized in that** the role of the applied low-intensity DC magnetic field, at least during the cooling down to a few kelvin, for example down to 10 K, is to detect a new transition of the pyrrhotite, called transition P.

7. Method according to one of Claims 1 to 3, **characterized in that** the magnetic transitions are also detected by monitoring the variation in the magnetic susceptibility of the specimen at low temperatures, down to a few degrees K.

## Patentansprüche

1. Verfahren zur Bestimmung der maximalen Temperatur, die eine Sedimentgesteinsprobe bei einer natürlichen Erwärmung, im Bereich von ungefähr 60 °C bis 230 °C, während eines geologischen Prozesses erfahren hat, **dadurch gekennzeichnet, dass** es die folgenden Schritte umfasst:
a) Untersuchen bei sehr niedrigen Temperaturen von bis zu einigen Kelvin, beispielsweise von bis zu 10 Kelvin, der magnetischen Übergänge, die dazu bestimmt sind, ein Eisenoxid und ein Eisensulfid zu charakterisieren, welche in der Probe durch die geologische Erwärmung des Sediments gebildet oder zerstört werden,
b) Bestimmen der maximalen Temperatur, welche die Probe erfahren hat, mit Hilfe einer Kalibrierung, die erzielt wurde, indem dieselben magnetischen Parameter an demselben Sediment oder anderen Sedimenten derselben Beschaffenheit nach vorherigem Erwärmen auf bekannte Temperaturen im Labor gemessen wurden.

2. Verfahren nach Anspruch 1, **dadurch gekennzeichnet, dass** es sich bei dem Eisenoxid und dem Eisensulfid um Magnetit und Pyrrhotin handelt.

3. Verfahren nach einem der Ansprüche 1 und 2, **dadurch gekennzeichnet, dass** es sich bei den gemessenen Übergängen um die magnetischen Übergänge handelt, die charakteristisch für Magnetit und Pyrrhotin sind und die bei unveränderlichen Temperaturen erfolgen, welche zwischen der ungefähren Raumtemperatur und einigen Kelvin, beispielsweise bis zu 10 Kelvin, erfolgen.

4. Verfahren nach einem der Ansprüche 1 bis 3, **dadurch gekennzeichnet, dass** die magnetischen Übergänge nachgewiesen werden, indem der Verlauf einer künstlichen isothermen remanenten Magnetisierung mitverfolgt wird, welche durch ein kontinuierliches Magnetfeld bewirkt wird, das ausreichend stark ist, um eine magnetische Sättigung mindestens des Magnetits und des Pyrrhotins zu bewirken, wenn eine solche möglich ist.

5. Verfahren nach einem der Ansprüche 1 bis 4, **dadurch gekennzeichnet, dass** ein kontinuierliches Magnetfeld von geringer Intensität, beispielsweise in der Größenordnung von 5 Mikrotesla, mindestens während des Abkühlens bis auf einige Kelvin, beispielsweise bis auf 10 K, zur Anwendung kommt.

6. Verfahren nach einem der Ansprüche 1 bis 5, **dadurch gekennzeichnet, dass** die Aufgabe des kontinuierlichen Magnetfeldes von geringer Intensität, das mindestens während des Abkühlens bis auf einige Kelvin, beispielsweise bis auf 10 K, zur Anwendung kommt, darin besteht, einen erneuten Übergang des Pyrrhotins nachzuweisen, welcher als P-Übergang bezeichnet wird.

7. Verfahren nach einem der Ansprüche 1 bis 3, **dadurch gekennzeichnet, dass** die magnetischen Übergänge weiterhin nachgewiesen werden, indem der Verlauf der magnetischen Suszeptibilität der Probe bei niedrigen Temperaturen bis zu einigen Grad K mitverfolgt wird.
